# EUROPÄISCHE PATENTSCHRIFT

(11) **EP 2 289 061 B1**
(45) Veröffentlichungstag und Bekanntmachung des Hinweises auf die Patenterteilung: **31.07.2019**
(21) Anmeldenummer: 09761752.6
(22) Anmeldetag: 10.06.2009
(51) Int. Cl.: G09B 23/28, A61B 19/00

(54) **OPHTHALMOSKOP-SIMULATOR**
OPHTHALMOSCOPE SIMULATOR
SIMULATEUR D'OPHTALMOSCOPE

(30) Priorität: 11.06.2008 DE 102008027832
(43) Veröffentlichungstag der Anmeldung: 02.03.2011
(73) Patentinhaber: VRmagic GmbH, 68167 Mannheim (DE)
(72) Erfinder: RUF, Thomas, 68165 Mannheim (DE); SCHILL, Markus, 69117 Heidelberg (DE); WAGNER, Clemens, Heidelberg 69115 (DE)
(74) Vertreter: Thews, Gustav
(86) Internationale Anmeldenummer: PCT/EP2009/057216
(87) Internationale Veröffentlichungsnummer: WO 2009/150190

(56) Entgegenhaltungen:
- EP-A2- 1 369 769
- WO-A1-99/60529
- US-B1- 6 847 336
- D. LEE, M WOO, D. VREDEVOE, J. KIMMICK, W.J. KARPLUS, D.J. VALENTINO: "Ophthalmoscopic Examination Training Using Virtual Reality" VIRTUAL REALITY, Bd. 4, Nr. 3, September 1999 (1999-09), Seiten 184-191, XP002553166 ISSN: 1359-4338 in der Anmeldung erwähnt

## Beschreibung

Die Erfindung bezieht sich auf ein Verfahren zum Betrieb eines Ophthalmoskop-Simulators zur Simulation der Handhabung eines Ophthalmoskops, aufweisend eine an einem Kopf eines Beobachters anbringbare Halterung (HMD), an der mindestens eine bildgebende erste Anzeige angeordnet ist, wobei die Anzeige vor einem Beobachterauge zwecks Darstellung einer virtuellen Realität platzierbar ist, wobei an der Halterung mindestens eine Videokamera zum Erzeugen von Videobildern der realen Umgebung vorgesehen ist, ein Positionserkennungssystem, über das die räumliche Position und/oder relative Lage der Halterung ermittelbar bzw. zu tracken ist, und einen Computer, über den von der mindestens einen Videokamera erzeugte Bilder der realen Umgebung auf der Anzeige dem Beobachter dargestellt werden können, wobei ein mittels einer Hand des Beobachters haltbarer Ophthalmoskop-Dummy und ein Patientenkopf-Dummy vorgesehen sind, und der Patientenkopf-Dummy zumindest eine einer Gesichtsform nachgebildete Peripheriefläche mit einer darin platzierten Patientenaugen-Position aufweist, auf der die Hand abgestützt werden kann, wobei mittels des Positionserkennungssystems die relative räumliche Position und Lage des Ophthalmoskop-Dummys und des Patientenkopf-Dummys ermittelbar ist, wobei in das dem Beobachter dargestellte Bild der realen Umgebung ein Bildteil einspielbar bzw. renderbar ist.

Es ist bereits ein Simulator zur Handhabung eines direkten Ophthalmoskops aus dem Aufsatz "Ophthalmoscopic Examination Training Using Virtual Reality" von D. Lee at al. (Springer Verlag London Ltd., Virtual Reality (1999) 4:184-191) bekannt. Der Simulationsaufbau besteht aus einer 3D-Maus, mittels welcher der Beobachter einen virtuellen Lichtkegel eines gedachten direkten Ophthalmoskops relativ zu einem auf einem Bildschirm dargestellten Patientenkopf bewegen kann. Alternativ ist eine 3D-Brille bzw. ein kopfgehaltenes Display vorgesehen, mittels dessen einem Beobachter der Patientenkopf einerseits sowie der durch ein Ophthalmoskop auf dem Patientenkopf erzeugbare Lichtkegel andererseits und eine Retina virtuell darstellbar sind. Die 3D-Maus oder das kopfgehaltene Display ist über ein Positionserkennungssystem getrackt, so dass entweder die relative Lage zwischen der Maus (dem Ophthalmoskop-Lichtkegel) und dem Bildschirm bzw. dem visualisierten Patientenkopf oder die absolute Lage des kopfgehaltenen Displays (dem Beobachter) erfassbar ist. Die virtuelle Szene wird in diesem Fall vollständig auf dem Display gerendert, ohne weiteren Referenzrahmen und ohne weitere handgehaltene Sensoren.

Die Würdigung dieses Aufsatzes in der hier prioritätsbegründenden DE 10 2008 027 832 A1 geht mithin über den Offenbarungsgehalt des Aufsatzes hinaus. Die Bestimmung der relativen Lage zwischen der Maus und dem kopfgehaltenen Display ist nicht vorgesehen. Deshalb wird zwecks Verbesserung der intuitiven Handhabung der Einsatz eines Ophthalmoskops vorgeschlagen (S. 190, Sp. 1, 2. Abs.). Alternativ wird vorgeschlagen, auf alle handgehaltenen Sensoren, also die Maus oder ein Ophthalmoskop zu verzichten und ein HMD einzusetzen, mit dem die Szene vollständig gerendert wird, ohne weiteren Referenzrahmen (S. 190, Sp. 1, 3. & 4. Abs.).

Die US 2005/0203367 A1 beschreibt ein interaktives Instrument zum Bedienen eines Darstellungssystems für CT-Daten, die in ein Bild einer realen Szene eingespielt werden. Das reale Bild ist das des Kopfes eines zu operierenden Patienten. Über das beschriebene System, bestehend aus HMD, Stift (pen) und Computer, werden die zur Verfügung gestellten CT-Daten in einer 3D-Darstellung dem realen Bild überlagert. Der Beobachter sieht also beides, das reale Bild und die virtuellen CT-Daten. Der Stift und das HMD werden getrackt, so dass mittels des Stiftes eine ebenfalls anzuzeigende interaktive Schaltfläche zum Bearbeiten der Darstellung der CT-Daten bedient werden kann. Die räumliche Anordnung der CT-Daten-Visualisierung erfolgt relativ zu sechs Punkten, die an dem fixierten Patientenkopf angeordnet sind. Die Punkte müssen zwecks Erfassung deren Position durch den Benutzer mittels des getrackten Stiftes angefahren werden. Hierzu muss die Reihenfolge bzw. die jeweilige Position der Punkte am Patientenkopf bekannt sein. Der Patientenkopf wird somit lediglich registriert. Die Punkte dienen sozusagen als fixe Anker für die CT-Daten Simulation. Sollte der Patientenkopf bewegt werden, würde die zwingend notwendige räumliche Korrelation zwischen den CT-Daten und dem Patientenkopf verloren gehen.

Die US 6,847,336 B1 beschreibt ein Assistenzsystem für den Operateur. Das Assistenzsystem weist mehrere Kameras auf, mittels derer Bilder der OP-Szene bzw. des Patienten aus anderen Blickwinkeln als der des Operateurs gemacht werden. Zudem ist ein semitransparenter Bildschirm vorgesehen, durch den der Operateur die reale OP-Szene betrachtet. Auf dem Bildschirm werden ergänzend die Bilder dargestellt, so dass es dem Operateur möglich ist, die OP-Szene, mithin sein Instrument, auch aus einem anderen Winkel zu betrachten. Zudem werden auf dem Bildschirm Auswahl- oder Schaltflächen angezeigt, die der Operateur durch Bewegung der Augen aktivieren kann. Über diese Auswahl- oder Schaltflächen kann der Operateur z. B. Vitaldaten des Patienten in unterschiedlicher Art anzeigen lassen.

Die EP 1 369 769 A2 beschreibt ein Verfahren zum Bestimmen der Genauigkeit der Registrierung bzw. Positionierung von Systemen zur Darstellung einer virtuellen Realität.

Die WO 99/60529 A1 beschreibt ebenfalls ein System zur Darstellung einer virtuellen Realität. Hiermit wird der virtuelle numerische Raum des Computers überlagert mit dem sensorischen realen Raum des Anwenders.

Der Erfindung liegt die Aufgabe zugrunde, ein Verfahren für einen Ophthalmoskop-Simulator derart auszubilden, dass eine möglichst realitätsnahe Simulation einer Augenuntersuchung gewährleistet ist.

Gelöst wird die Aufgabe erfindungsgemäß dadurch, dass
mittels des Positionserkennungssystems die räumliche Position und Lage des Ophthalmoskop-Dummys und des Patientenkopf-Dummys und der Halterung ermittelt werden,
und in Abhängigkeit der relativen Lage dieser drei Objekte ein von der mindestens einen Videokamera erzeugtes Bild der realen Umgebung betreffend den Patientenkopf-Dummy und den Ophthalmoskop-Dummy auf der Anzeige dargestellt wird, und
in dem dargestellten Bild der realen Umgebung an die Stelle des Ophthalmoskop-Dummys der Bildteil eingespielt wird, wobei der eingespielte Bildteil eine virtuelle Retina und/oder eine virtuelle Umgebung darstellt, wie sie der Beobachter durch das von ihm gehaltene Ophthalmoskop sehen würde, wenn es sich um ein entsprechendes Instrument handeln würde,
wobei der eingespielte Bildteil die virtuelle Retina nur dann darstellt, wenn die relative Lage zwischen der Halterung und dem Ophthalmoskop-Dummy und dem Patientenkopf-Dummy vordefinierte realitätsnahe Bedingungen erfüllt, und dass es sich bei dem Ophthalmoskop-Dummy um einen Dummy für eine Linse eines indirekten Ophthalmoskops oder einen Dummy für ein direktes Ophthalmoskop handelt.

Wenn ein Bild eines virtuellen Patientenkopfes dargestellt wird, dann wird dieses mit dem Bild des Ophthalmoskop-Dummys räumlich in Übereinstimmung gebracht. Der Ophthalmoskop-Dummy, der Patientenkopf-Dummy und die Halterung bzw. das HMD werden also im Betrieb zwecks Berücksichtigung der relativen Lage zueinander getrackt.

Erfindungswesentlich ist, dass neben der die Lage bzw. Position des Beobachterkopfes wiedergebenden Halterung ein Dummy für das Ophthalmoskop bzw. die Ophthalmoskoplinse sowie ein Dummy für einen Patientenkopf vorgesehen werden, wobei über das Positionierungssystem die räumliche Lage der drei zuvor genannten Gegenstände relativ zueinander zu jedem Zeitpunkt bekannt ist. Über die Anzeigen wird dem Beobachter, in Abhängigkeit seiner eigenen Kopfposition, die sich vor ihm ergebende Umgebung dargestellt, wobei die Position des von ihm gehaltenen Dummys des Ophthalmoskops sowie die Position des Patientenkopf-Dummys andererseits angezeigt bzw. eingespielt werden. Dabei kann sowohl die Umgebung als auch der Patientenkopf sowie der Ophthalmoskop-Dummy auf beliebige Weise visualisiert bzw. dargestellt werden, z. B. als reales Bild oder als virtuelles Bild. Das virtuelle Bild des Patientenkopfes würde räumlich mit dem realen Bild des Patientenkopf-Dummys in Übereinstimmung gebracht werden. Den Ophthalmoskop-Dummy betreffend, wird im Falle einer Ophthalmoskoplinse ein Bildteil eingespielt, der sich ergäbe, wenn der Beobachter in Realität durch eine Ophthalmoskoplinse schauen würde. Er sieht also vorzugsweise die hinter der Linse vorhandene Umgebung stark vergrößert und auf den Kopf gestellt. Nur in dem Fall, in dem er den Ophthalmoskoplinsen-Dummy in die richtige relative Lage zwischen der Halterung und dem Patientenkopf-Dummy bringt, wird ihm als Bildteil ein virtuelles Bild einer Retina eingespielt, wie er sie in Realität sehen würde.

Das Positionserkennungssystem weist im Allgemeinen eine Basis auf, die entweder an der Halterung oder an dem Patientenkopf-Dummy platziert ist. Alternativ kann sie auch separat im Raum platziert sein. In den ersten beiden Fällen ist die zu rendernde Szene erheblich kleiner, was eine höheren Tracking-Geschwindigkeit bzw. Qualität erlaubt. Die Positionserkennung erfolgt in der Regel durch ein oder mehrere Marker, die an dem zu erkennenden Objekt, also dem Ophthalmoskop-Dummy oder dem Patientenkopf-Dummy, angebracht sind. Das Positionserkennungssystem ist vorzugsweise optisch, d.h. die Basis weist entsprechende Kameras bzw. Videokameras auf, mittels denen die Marker bzw. deren Position ausgewertet erfasst werden. Mittels komplexerer Positionserkennungssysteme lassen sich vorgenannte Objekte auch ohne das Anbringen entsprechender Marker erkennen. Durch den
Einsatz von Markern wird das System jedoch wesentlich ein-facher, insbesondere für die Erkennung des Patientenkopf-Dummys bzw. der Peripheriefläche um die Augenposition her-um, die vorzugsweise einer Gesichtsform nach ausgebildet ist. Die Basis verfügt meist über zwei Kameras, aber der Einsatz einer Kamera zum Erfassen der Marker zwecks an-schließender Auswertung deren Position, also zum Tracken, ist auch möglich.

An der Halterung ist mindestens eine Videokamera zum Erzeugen von Videobildern der realen Umgebung vorgesehen und durch das Videobild ist zumindest ein Teil der Umgebung, wie der Patientenkopf-Dummy, die Hand des Beobachters, der Ophthalmoskop-Dummy und/oder ein Teil davon auf der Anzeige darstellbar. Hierbei ist über das Positionserkennungssystem die räumliche Position und/oder Lage der Videokamera zumindest mittelbar über die Halterung erfassbar. Die Videokamera ist ergänzend zu den Videokameras des Positionserkennungssystems vorgesehen.

Das dem Beobachter dargestellte Bild setzt sich in diesem Fall aus Bildern der realen, sich vor dem Beobachter er-streckenden Umgebung sowie eingespielten Bildteilen zusammen. Die reale Umgebung betrifft neben dem vor ihm positionierten Patientenkopf-Dummy die eigene Hand, mit der er den Ophthalmoskop-Dummy hält, den Ophthalmoskop-Dummy sowie die sich außen herum ergebende Laboratmosphäre. Der eingespielte Bildteil bezieht sich auf das, was der Beobachter durch die von ihm gehaltene Ophthalmoskoplinse bzw. das Ophthalmoskop sehen würde, wenn es sich um ein entsprechendes Instrument handeln würde. Im Falle eines Ophthalmoskoplinsen-Dummys wird dem Beobachter über das Display gerade noch der Rand des von ihm gehaltenen Dummys dargestellt, wobei der
Bildteil innerhalb, also dort, wo sich bei einer Ophthalmoskoplinse der Glaskörper befindet, eine entsprechend vergrößerte und auf dem Kopf stehende Abbildung dessen dargestellt wird, was sich hinter dem Ophthalmoskoplinsen-Dummy real oder virtuell befindet. Dies können auch Teile der Umgebung oder des Patientenkopf-Dummys sein. In dem Fall, in dem die relative Lage zwischen dem Beobachterkopf, also der Halterung und dem Ophthalmoskop-Dummy sowie dem Patientenkopf-Dummy bezüglich der Winkel und Abstände eine vorbestimmte, realitätsnahe Bedingung erfüllt, wird dem Beobachter als Bildteil das Bild einer virtuellen Retina, wie sie sich in der Praxis ergeben würde, angezeigt. Ergänzend können auch der Rand der virtuellen Ophthalmoskoplinse und/oder ein virtueller Patientenkopf einspielbar sein.

Hierbei kann vorgesehen sein, dass der Ophthalmoskop-Dummy ein Dummy für eine Linse eines indirekten Ophthalmoskops oder ein Dummy für ein direktes Ophthalmoskop ist. Im Falle eines Dummys für eine Ophthalmoskoplinse kann der Beobachter, wie im Falle einer echten Untersuchung, die Hand, mit der er den Ophthalmoskoplinsen-Dummy hält, auf der Peripheriefläche um die Augenposition am Patientenkopf-Dummy abstützen. In dem anderen Fall, also bei einem Dummy für ein direktes Ophthalmoskop, wird er diesen unmittelbar vor sein eigenes Auge bzw. vor die Anzeige bzw. das Head-Mounted Display (HMD) halten. Der realitätsnahe taktile Bezug zum Patientenkopf bzw. Patientenkopf-Dummy besteht in diesem Fall nicht.

Ferner kann es vorteilhaft sein, wenn die Halterung, die Videokamera, der Ophthalmoskop-Dummy, der Patientenkopf-Dummy, die Patientenaugen-Position und/oder die Peripheriefläche jeweils mindestens einen, vorzugsweise drei Marker aufweisen, mittels derer über das Positionserkennungssystem die räumliche Position und/oder Lage relativ zum Positionserkennungssystem ermittelbar ist. Der Einsatz von einem oder mehreren Markern vereinfacht die Erkennung der Lage bzw. Position der damit markierten Objekte. Eine freie Positionserkennung ohne den Einsatz von Markern ist ebenfalls möglich, aber deutlich aufwendiger. Mit der Positionserkennung der Halterung bzw. des Head-Mounted Displays ist die Position bzw. relative Lage des Beobachterkopfes bzw. der Beobachteraugen ebenfalls bekannt. Zusätzliche Marker für den Beobachterkopf sind somit nicht notwendig, können jedoch vorgesehen sein. Der Patientenkopf-Dummy weist vorzugsweise mehr als drei, jedoch maximal 37 Marker auf. Durch den Einsatz von deutlich mehr Markern lässt sich der Patientenkopf-Dummy, der vorzugsweise eine gesichtsähnliche Form aufweist, optimal erkennen bzw. seine relative Lage optimal bestimmen.

Daneben kann es vorteilhaft sein, wenn eine zweite Anzeige vorgesehen ist, wobei die erste Anzeige vor dem rechten Auge und die zweite Anzeige vor einem linken Auge des Beobachters platzierbar ist und wobei ein Abstand a4 zwischen den Anzeigen einstellbar ist und dass eine zweite Videokamera vorgesehen ist, wobei das Videobild der zweiten Videokamera über die zweite Anzeige darstellbar ist und wobei ein Abstand a5 zwischen den Videokameras einstellbar ist. Somit lassen sich sowohl der Augenabstand der Displays als auch der Abstand der stereoskopischen Basis beliebig einstellen. Die zweite Videokamera ist ebenfalls ergänzend zu den Kameras des Positionserkennungssystems vorgesehen.

Zudem kann es vorteilhaft sein, wenn Mittel zum Simulieren eines Lichtfilters vorgesehen sind. Damit lassen sich realitätsnahe Bedingungen für den Beobachter schaffen.

Vorteilhafterweise kann vorgesehen sein, dass Mittel zur Darstellung eines Abstandes a1, a2 und/oder a3 und/oder Mittel zur Darstellung einer Abweichung des Abstandes a1, a2 und/oder a3 von einem Sollwert vorgesehen sind, wobei der Abstand a1 der Abstand zwischen der Halterung und der Patientenaugen-Position, der Abstand a2 der Abstand zwischen dem Ophthalmoskop-Dummy und der Patientenaugen-Position und der Abstand a3 der Abstand zwischen der Halterung und dem Ophthalmoskop-Dummy ist. Für ungeübte Beobachter ist es sehr schwierig die richtige relative Lage des Ophthalmoskops bzw. der Ophthalmoskoplinse bzw. des jeweiligen Dummys zu finden, um das gewünschte Bild der Retina zu erhalten. Die Anzeige vorstehend genannter Abstände soll dem trainierenden Beobachter helfen, die richtige relative Lage zu erreichen und während der Untersuchung eine Kontrolle dieser Lage zu erhalten. Ausgehend von der Position der Halterung kann die Position des Beobachter-Auges oder anderer Teile der Halterung berechnet und im Abstand a1, a3 berücksichtigt werden.

Vorteilhaft kann es ferner sein, wenn die Mittel optische Anzeigemittel umfassen, die über die Anzeige erkennbar sind und dass der Computer Mittel zum Speichern, Abrufen, Auswerten und/oder Verändern der Bilder und/oder der Bildteile und zum Erstellen einer Zusammenschau der Bereiche aufweist, die der Beobachter auf der virtuellen Retina während einer simulierten Untersuchung beobachtet hat. Insbesondere die Zusammenschau bzw. Aufstellung der Bereiche, die der Beobachter während der simulierten Untersuchung gesehen hat, gibt ihm einen äußerst wichtigen Eindruck über die bisherige Handhabung und den Beobachtungspfad den er auf der Retina vollzogen hat.

Ergänzend kann es vorteilhaft sein, wenn die im Computer gespeicherten oder veränderten Bilder, Bildteile und/oder Videobilder auf der Anzeige oder einem dafür vorgesehenen separaten Monitor darstellbar sind. Somit ist es einer weiteren Person möglich, den optischen Eindruck des Beobachters zeitgleich nachzuvollziehen.

Verfahrensseitig wird dabei der Bildteil, der die Retina darstellt, nur dann eingespielt, wenn die relative Lage zwischen der Halterung, dem Ophthalmoskop-Dummy und dem Patientenkopf-Dummy vordefinierte Bedingungen erfüllt. Erst wenn der Beobachter den Ophthalmoskop-Dummy so platziert, wie es in Realität mit einer Ophthalmoskoplinse oder einem direkten Ophthalmoskop notwendig wäre um die Retina sehen zu können, wird der ent-sprechende Bildteil eingespielt. Bei der Auswahl des die Retina darstellenden Bildteils wird die relative Lage des Ophthalmoskop-Dummys berücksichtigt. Hierzu kann ein entsprechender Fundus von Bildteilen der Retina vorgesehen sein.

Verfahrensseitig wird dabei mittels der Videokamera das Videobild der realen Umgebung, wie der Patientenkopf-Dummy, die Hand des Beobachters, der Ophthalmoskop-Dummy und/oder einen Teil davon erfasst und auf der Anzeige dargestellt. Hieraus ergeben sich die oben genannten Vorteile betreffend den Aufbau des dem Beobachter dargestellten Bildes, zusammengesetzt aus der realen, vor ihm existierenden Umgebung einerseits sowie den eingespielten Bildteilen, virtueller Teile, andererseits.

Entsprechend kann es von Vorteil sein, wenn ein Abstand a1 zwischen der Halterung und der Patientenaugen-Position und/oder der Abstand a2 zwischen dem Ophthalmoskop-Dummy
und der Patientenaugen-Position und/oder der Abstand a3 zwischen der Halterung und dem Ophthalmoskoplinsen-Dummy und/oder eine Abweichung des jeweiligen Abstandes a1, a2, a3 von einem Sollwert dem Beobachter angezeigt wird. Ergänzend oder alternativ können auch andere Parameter der relativen Lage zwischen der Halterung bzw. dem Beobachter Kopf, dem Ophthalmoskop-Dummy und/oder dem Patientenkopf-Dummy, wie z.B. verschiedene Kippwinkel des Ophthalmoskop-Dummys oder der Blickwinkel des Beobachters, oder deren Abweichung von einem Sollwert in entsprechender Weise angezeigt werden. Bei dem Kippwinkel handelt es sich insbesondere um Dreh- bzw. Schwenkwinkel um eine Achse rechtwinklig zur optischen Achse des simulierten Ophthalmoskop-Dummys oder der Optik.

Ergänzend kann es von Vorteil sein, wenn für die Lage und/oder Bewegung der Halterung und/oder des Ophthalmoskop-Dummys Bilder berechnet werden, die eine Ansicht aus mindestens einem anderen Blickwinkel im Raum wiedergeben. Somit sind die von dem Beobachter ausgeführten Bewegungen von einer anderen Position im Raum, vorzugsweise einer Position seitlich zum Beobachter darstellbar. Der Beobachter kann im Nachhinein einen Eindruck von seinem Positions- und Bewegungsverhalten erhalten.

Vorteilhaft kann es auch sein, wenn Augenbewegungen des Patienten simuliert werden. Da Patienten oftmals Augenbewegungen durch Verändern der Blickrichtung oder durch Lidschlag ausführen, kann es zwecks Training hilfreich sein, solche Bewegungen auch zu simulieren.

Vorteilhaft ist auch ein Computersystem oder Datenträger bzw. ein Computerprogramm zum Ausführen des vorstehend beschriebenen Verfahrens.

Weitere Vorteile und Einzelheiten der Erfindung sind in den Patentansprüchen und in der Beschreibung erläutert und in den Figuren dargestellt. Es zeigt:
- Figur 1: eine Prinzipskizze eines Ophthalmoskop-Simulators für ein indirektes Ophthalmoskop;
- Figur 2a: ein dargestelltes Bild einer Umgebung mit Patientenkopf-Dummy und Ophthalmoskoplinsen-Dummy;
- Figur 2b: ein dargestelltes Bild nach Fig. 2a mit eingespieltem Bildteil einer Retina;
- Figur 2c: ein dargestelltes Bild nach Fig. 2b mit eingespieltem Bildteil der Umgebung;
- Figur 3: ein Dummy eines direkten Ophthalmoskops.

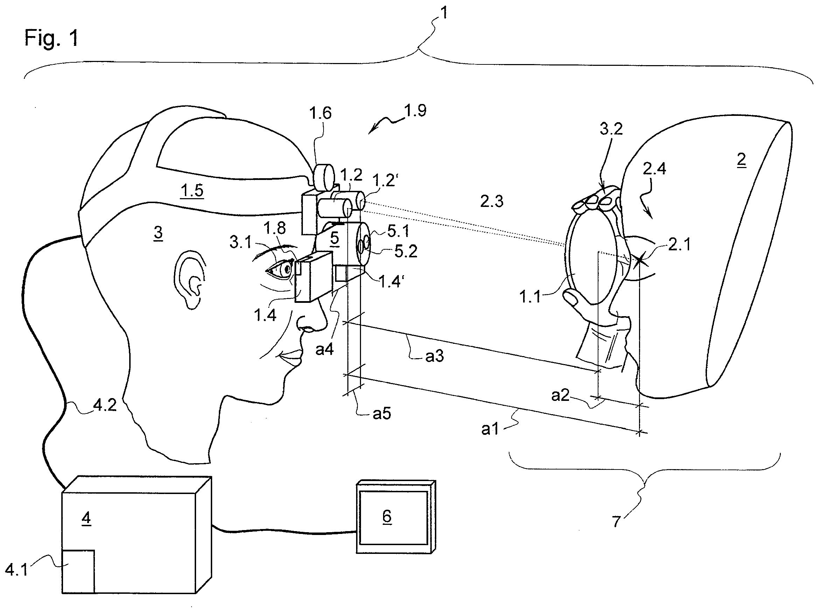

Der in Figur 1 dargestellte Ophthalmoskop-Simulator 1 besteht aus einer Halterung 1.5, die auf einem Beobachterkopf 3 aufsetzbar ist und an der im Wesentlichen eine erste Anzeige 1.4 und eine zweite Anzeige 1.4' angeordnet sind. Den Anzeigen 1.4, 1.4' sind zwei Videokameras 1.2, 1.2' zugeordnet, die die Szene erfassen. Eine solche Vorrichtung wird auch als Head-Mounted Display (HMD) 1.9 bezeichnet. Das HMD 1.9 weist zusätzlich eine Basis 5 eines Positionserkennungssystems mit zwei Videokameras 5.1, 5.2 als Grundlage zum optischen Tracken auf. Das so ausgerüstete HMD 1.9 ist über eine Verbindungsleitung 4.2 mit einem Computer 4 bzw. einer Bildbearbeitungseinheit verbunden. Die Bildbearbeitungseinheit 4 weist Mittel 4.1 zum Speichern, Abrufen, Auswerten und/oder Verändern von Bildern 1.3 oder Bildteilen 2.2 sowie zur Erstellung einer Zusammenschau der verschiedenen Bilder 1.3, 2.2 auf. Ferner ist mit dem Computer 4 ein Monitor 6 verbunden, auf dem die auf den Anzeigen 1.4, 1.4' dargestellten Bilder 1.3 ebenfalls dargestellt werden können. Es ist auch möglich weitere Bilder, wie eine Ansicht aus einem anderen Blickwinkel im Raum darzustellen.

Das HMD 1.9 ist auf den Kopf 3 des Beobachters aufgesetzt, so dass die beiden Anzeigen 1.4, 1.4' bzw. die beiden Bildschirme vor dem jeweiligen Auge 3.1 des Beobachters positioniert sind. Hierbei ist ein Abstand a4 zwischen den beiden Bildschirmen 1.4, 1.4' zwecks Anpassung an den Augenabstand variabel einstellbar.

Der Ophthalmoskop-Simulator 1 weist zudem einen Ophthalmoskoplinsen-Dummy 1.1 auf, der mittels der Hand des Beobachters 3.2 gehalten bzw. positioniert wird. Ein weiterer Teil des Ophthalmoskop-Simulators 1 ist ein Patientenkopf-Dummy 2, der die Form eines Kopfes bzw. eines Gesichtes hat. Der Patientenkopf-Dummy 2 weist eine als Augenhöhle ausgebildete Position 2.1 eines zu untersuchenden Patientenauges sowie eine Peripheriefläche 2.4 auf, innerhalb derer die Position 2.1 vorgesehen ist.

Der Ophthalmoskoplinsen-Dummy 1.1 kann, wie eine Ophthalmoskoplinse eines indirekten Ophthalmoskops, mittels der Hand 3.2 des Beobachters vor dem zu beobachtenden Patientenauge bzw. der Position 2.1 platziert werden. Das HMD 1.9 stellt insoweit den kopfgehaltenen Teil eines indirekten Ophthalmoskops dar, bei dem über eine entsprechende Halterung 1.5 eine Beobachteroptik vor dem Beobachterauge 3.1 positioniert wird.

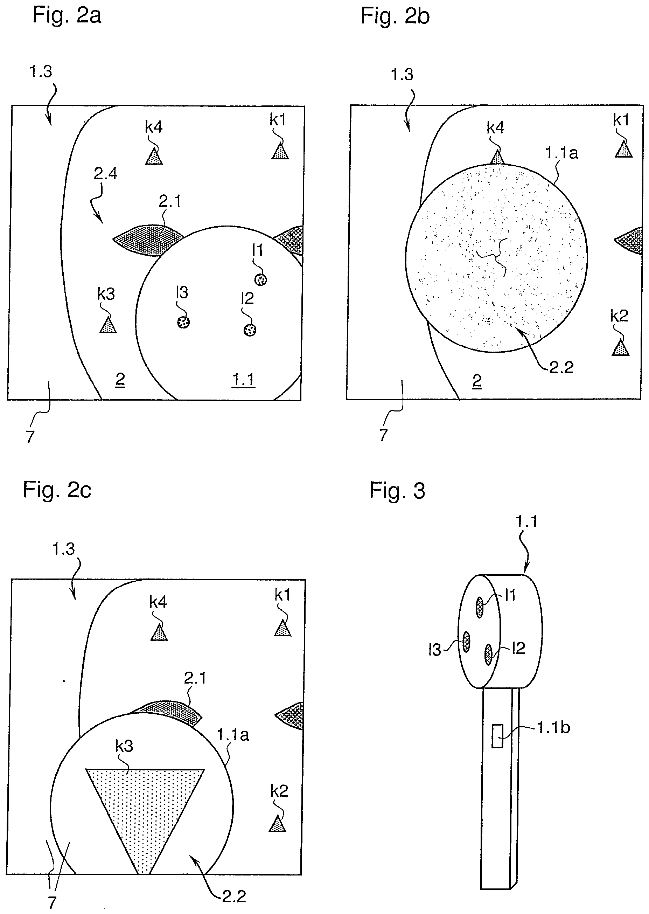

Das über die beiden Videokameras 1.2, 1.2' erhaltene Bild 1.3 des Ophthalmoskoplinsen-Dummys 1.1 einerseits sowie des Patientenkopf-Dummys 2 und dessen Umgebung 7 andererseits, werden dem Beobachter über die Bildschirme 1.4, 1.4' in einer Art gemäß Figuren 2a bis 2c dargestellt. Über die Basis des Positionserkennungssystems 5 wird die relative Lage des Ophthalmoskoplinsen-Dummys 1.1 und des Patientenkopf-Dummys 2 zum HMD 1.9 ermittelt und kann somit virtuell dargestellt bzw. eingespielt werden.

Innerhalb der Anzeige 1.4, 1.4' sind optische Anzeigemittel 1.8 vorgesehen, die dem Beobachter unter anderem die bezeichneten Abstände a1 bis a3 und ggf. eine Abweichung davon anzeigen. Die optischen Anzeigemittel 1.8 sind vorzugsweise ein Teil der als Display ausgebildeten Anzeige 1.4, 1.4'. Gesonderte Anzeigemittel 1.8 sind in der Regel nicht notwendig. Ferner sind am HMD 1.9 weiter Mittel 1.6 zum Simulieren von optischen Filtern während der Untersuchung vorgesehen.

Das dem Beobachter dargestellte Bild gemäß Fig. 2a besteht aus dem realen von den Videokameras 1.2, 1.2' erfassten Bild der Umgebung, also dem Patientenkopf-Dummy 2 und dem Ophthalmoskoplinsen-Dummy 1.1. Auf eine Darstellung der Hand 3.2 des Beobachters sowie anderer Gegenstände aus der weiteren Umgebung 7 wurde zwecks Übersichtlichkeit verzichtet. In dem, dem Beobachter dargestellten Bild bzw. Videobild 1.3, sind die verschiedenen Marker k1, k3 und k4 des Patientenkopf-Dummys 2 sowie die Patientenaugen-Position 2.1 zu sehen. Ferner sind die Marker l1 bis l3 des Ophthalmoskoplinsen-Dummys 1.1 zu sehen. Seitlich zum Patientenkopf-Dummy 2 erstreckt sich die Umgebung 7. Die Marker sind in einer bevorzugten Ausführungsform nicht zu erkennen und befinden sich unterhalb einer nicht dargestellten Deckschicht.

Sobald der Beobachter den Ophthalmoskoplinsen-Dummy 1.1 in die richtige Position vor das soweit simulierte Patientenauge bzw. die Patientenaugen-Position 2.1 bringt, wird an die Stelle des Ophthalmoskoplinsen-Dummys 1.1 ein Bildteil 2.2 eingespielt, der ein virtuelles Bild der Retina, wie sie dem Beobachter erscheinen würde, darstellt. Das Bild des Ophthalmoskoplinsen-Dummys 1.1 wird hierbei großteils durch den Bildteil 2.2 ersetzt, wobei vorzugsweise ein Rand 1.1a des Ophthalmoskoplinsen-Dummys 1.1 aus dem realen Bild 1.3 der Videokameras 1.2, 1.2' verbleibt.

Vorzugsweise ist es ebenfalls vorgesehen, einen entsprechenden Bildteil 2.2 auch dann an die Stelle des Ophthalmoskoplinsen-Dummys 1.1 einzuspielen, wenn der Ophthalmoskoplinsen-Dummy 1.1 nicht in einer die Retina abbildenden Position gehalten wird. Dies ist in Fig. 2c dargestellt. Der Ophthalmoskoplinsen-Dummy 1.1 ist in diesem Fall im Bereich des Markers k3 positioniert und bildet diesen vergrößert und auf dem Kopf stehend für den Beobachter ab. Demnach ist eine reale Darstellung des Ophthalmoskoplinsen-Dummys 1.1 gemäß Fig. 2a nicht zwingend notwendig. Dem Beobachter kann anstelle des Ophthalmoskoplinsen-Dummys 1.1 grundsätzlich der entsprechende reale oder virtuelle Bildteil 2.2, sei es von der Umgebung 7 oder bei korrekter Ausrichtung des Ophthalmoskoplinsen-Dummys 1.1, der entsprechende Bildteil 2.2 der Retina dargestellt werden.

In einer weiteren Ausführungsform ist es auch vorgesehen, anstatt der Simulation eines indirekten Ophthalmoskops, bei der der Beobachter einen Ophthalmoskoplinsen-Dummy 1.1 handgehalten führt, ein direktes Ophthalmoskop in Form eines entsprechenden Dummys 1.1 gemäß Fig. 3 zu simulieren.

Der Dummy 1.1 des direkten Ophthalmoskops weist entsprechende Marker l1 bis l3 auf, die eine Erkennung der Position bzw. Lage im Raum ermöglichen. Ferner weist der Dummy 1.1 weitere taktile Mittel 1.1b auf, durch die eine Simulation von Schaltern bzw. weiteren Bedienelementen eines direkten Ophthalmoskops möglich ist.

### Bezugszeichenliste

- 1: Ophthalmoskop-Simulator
- 1.1: Ophthalmoskoplinsen-Dummy
- 1.1a: Rand
- 1.1b: taktile Mittel
- 1.2: Videokamera
- 1.2': zweite Videokamera
- 1.3: Bild, Videobild
- 1.4: erste Anzeige, Bildschirm
- 1.4': zweite Anzeige, Bildschirm
- 1.5: Halterung
- 1.6: Mittel
- 1.8: Mittel, optische Anzeigemittel
- 1.9: Head Mounted Display (HMD)
- 2: Patientenkopf-Dummy
- 2.1: Patientenaugen-Position
- 2.2: Bildteil, Bild der Retina, Bild der Umgebung
- 2.4: Peripheriefläche
- 3: Kopf
- 3.1: Beobachterauge, rechtes Auge
- 3.2: Hand des Beobachters
- 4: Computer, Bildbearbeitungseinheit
- 4.1: Mittel
- 4.2: Verbindungsleitung
- 5: Positionserkennungssystem, Basis
- 5.1: Videokamera
- 5.2: Videokamera
- 6: Monitor
- 7: Umgebung
- a1 - a5: Abstand
- k1 - k4: Marker
- l1 - l3: Marker

## Patentansprüche

1. a Verfahren zum Betrieb eines Ophthalmoskop-Simulators (1) zur Simulation der Handhabung eines Ophthalmoskops, aufweisend
b eine an einem Kopf (3) eines Beobachters anbringbare Halterung (1.5), an der mindestens eine bildgebende erste Anzeige (1.4) angeordnet ist,
b1 wobei die Anzeige (1.4) vor einem Beobachterauge (3.1) zwecks Darstellung einer virtuellen Realität positionierbar ist,
b2 wobei an der Halterung (1.5) mindestens eine Videokamera (1.2) zum Erzeugen von Videobildern (1.3) der realen Umgebung vorgesehen ist,
c ein Positionserkennungssystem (5), über das die räumliche Position und Lage der Halterung (1.5) ermittelbar ist,
d und einen Computer (4), über den von der mindestens einen Videokamera erzeugte Bilder (1.3) der realen Umgebung (7) auf der Anzeige (1.4) dem Beobachter dargestellt werden können,
e wobei ein Ophthalmoskop-Dummy (1.1) und ein Patientenkopf-Dummy (2) vorgesehen sind,
e1 und der Patientenkopf-Dummy (2) zumindest eine einer Gesichtsform nachgebildeten Peripheriefläche (2.4) mit einer darin platzierten Patientenaugen-Position (2.1) aufweist, auf der die Hand abgestützt werden kann,
f wobei mittels des Positionserkennungssystems (5) die räumliche Position und Lage des Ophthalmoskop-Dummys (1.1) und des Patientenkopf-Dummys (2) ermittelbar ist
g wobei in das dem Beobachter dargestellte Bild (1.3) der realen Umgebung (7) ein Bildteil (2.2) einspielbar ist,
h wobei
- mittels des Positionserkennungssystems (5) die räumliche Position und Lage des Ophthalmoskop-Dummys (1.1) und des Patientenkopf-Dummys (2) und der Halterung (1.5) ermittelt werden,
i und in Abhängigkeit der relativen Lage dieser drei Objekte (1.1, 1.2, 1.5)
i1 - ein von der mindestens einen Videokamera erzeugtes Bild (1.3) der realen Umgebung (7) betreffend den Patientenkopf-Dummy und den Ophthalmoskop-Dummy (1.1) auf der Anzeige (1.4) dargestellt wird, und
i2 - in dem dargestellten Bild (1.3) der realen Umgebung (7) an die Stelle des Ophthalmoskoplinsen-Dummys (1.1) der Bildteil (2.2) eingespielt wird, wobei der eingespielte Bildteil (2.2) eine virtuelle Retina und/oder eine virtuelle Umgebung (7) darstellt, wie sie der Beobachter durch das von ihm gehaltene Ophthalmoskop sehen würde, wenn es sich um ein entsprechendes Instrument handeln würde,
i3 wobei
- der eingespielte Bildteil (2.2) die virtuelle Retina nur dann darstellt, wenn die relative Lage zwischen der Halterung (1.5) und dem Ophthalmoskop-Dummy (1.1) und dem Patientenkopf-Dummy (2) vordefinierte realitätsnahe Bedingungen erfüllt;
k wobei es sich bei dem Ophthalmoskop-Dummy (1.1) um einen Dummy für eine Linse (1.1) eines indirekten Ophthalmoskops oder einen Dummy für ein direktes Ophthalmoskop handelt.

2. Verfahren nach Anspruch 1,
**dadurch gekennzeichnet,**
**dass** mittels der Videokamera (1.2) das Videobild (1.3) der realen Umgebung (7), wie der Patientenkopf-Dummy (2), die Hand (3.2) des Beobachters, der Ophthalmoskop-Dummy (1.1) und/oder ein Teil davon erfasst und auf der Anzeige (1.4) dargestellt wird.

3. Verfahren nach Anspruch 1 oder 2,
**dadurch gekennzeichnet,**
**dass** ein Abstand a1 zwischen der Halterung (1.5) und der Patientenaugen-Position (2.1) und/oder der Abstand a2 zwischen dem Ophthalmoskop-Dummy (1.1) und der Patientenaugen-Position (2.1) und/oder der Abstand a3 zwischen der Halterung (1.5) und dem Ophthalmoskoplinsen-Dummy (1.1) und/oder eine Abweichung des jeweiligen Abstandes a1, a2, a3 von einem Sollwert dem Beobachter angezeigt wird.

4. Verfahren nach einem der Ansprüche 1 bis 3,
**dadurch gekennzeichnet,**
**dass** für die Lage und/oder Bewegung der Halterung (1.5) und/oder des Ophthalmoskop-Dummys (1.1) Bilder berechnet werden, die eine Ansicht aus mindestens einem anderen Blickwinkel im Raum wiedergeben.

5. Verfahren nach einem der Ansprüche 1 bis 4,
**dadurch gekennzeichnet,**
**dass** Augenbewegungen des Patienten simuliert werden.

## Claims

1. a A method of operating an ophthalmoscope simulator (1) for simulating the manual manipulation of an ophthalmoscope, comprising
b a support (1.5) which can be attached to the head (3) of an observer, on which support at least one first imaging display (1.4) is arranged,
b1 wherein the display (1.4) can be positioned in front of one eye (3.1) of the observer for the purposes of representing a virtual reality,
b2 wherein at least one video camera (1.2) for generating video images (1.3) of the real surroundings is provided on the support (1.5),
c a position recognition system (5) whereby the spatial position and relative position of the support (1.5) can be determined,
d and a computer (4) on which images (1.3) of the real surroundings (7) generated by the at least one video camera can be displayed to the observer on the display (1.4),
e wherein an ophthalmoscope dummy (1.1) and a patient head dummy (2) are provided,
e1 and the patient head dummy (2) has at least one peripheral surface (2.4) which replicates a facial shape and has a patient eye position (2.1) located therein, on which the hand can be supported,
f wherein the spatial position and relative position of the ophthalmoscope dummy (1.1) and of the patient head dummy (2) can be determined by means of the position recognition system (5),
g wherein an image component (2.2) can be incorporated into the image (1.3) of the real surroundings (7) displayed to the observer,
h wherein
- the spatial position and relative position of the ophthalmoscope dummy (1.1) and of the patient head dummy (2) and the support (1.5) are determined by means of the position recognition system (5),
i and as a function of the relative position of these three objects (1.1, 1.2, 1.5)
i1 an image (1.3) of the real surroundings (7) generated by the at least one video camera and concerning the patient head dummy and the ophthalmoscope dummy (1.1) is displayed on the display (1.4), and
i2 the image component (2.2) is incorporated into the displayed image (1.3) of the real surroundings (7), instead of the ophthalmoscope lens dummy (1.1), wherein the incorporated image component (2.2) represents a virtual retina and/or virtual surroundings (7) as they would be seen by the observer through the ophthalmoscope held by him if it were an appropriate instrument,
i3 wherein
- the incorporated image component (2.2) only represents the virtual retina if the relative position between the support (1.5) and the ophthalmoscope dummy (1.1) and the patient head dummy (2) satisfies predetermined realistic conditions;
k wherein the ophthalmoscope dummy (1.1) is a dummy for a lens (1.1) of an indirect ophthalmoscope or a dummy for a direct ophthalmoscope.

2. The method according to Claim 1,
**characterised in that**
the video image (1.3) of the real surroundings (7), such as the patient head dummy (2), a hand (3.2) of the observer, the ophthalmoscope dummy (1.1) and/or a part of the same, are captured by means of the video camera (1.2) and displayed on the display (1.4).

3. The method according to Claim 1 or 2,
**characterised in that**
a distance a1 between the support (1.5) and the patient eye position (2.1) and/or the distance a2 between the ophthalmoscope dummy (1.1) and the patient eye position (2.1) and/or the distance a3 between the support (1.5) and the ophthalmoscope lens dummy (1.1) and/or a deviation of the respective distance a1, a2, a3 from a nominal value is/are displayed to the observer.

4. The method according to any one of Claims 1 to 3,
**characterised in that**
images are computed for the position and/or movement of the support (1.5) and/or of the ophthalmoscope dummy (1.1), which images reproduce a view from at least one other viewpoint in the space.

5. The method according to any one of Claims 1 to 4,
**characterised in that**
eye movements of the patient are simulated.

## Revendications

1. a Procédé pour l'exploitation d'un simulateur d'ophtalmoscope (1) pour la simulation de la manipulation d'un ophtalmoscope, comprenant
b une fixation (1.5) pouvant être montée sur la tête (3) d'un observateur, sur laquelle fixation au moins un premier dispositif d'affichage (1.4) d'imagerie est disposé,
b1 le dispositif d'affichage (1.4) pouvant être positionné devant l'oeil d'un observateur (3.1) aux fins de la représentation d'une réalité virtuelle,
b2 au moins une caméra vidéo (1.2) étant prévue sur la fixation (1.5) pour la génération d'images vidéo (1.3) de l'environnement réel,
c un système de reconnaissance de position (5) par lequel la position spatiale et la situation de la fixation (1.5) peuvent être déterminées,
d et un ordinateur (4), par lequel des images (1.3) de l'environnement réel (7) générées par l'au moins une caméra vidéo peuvent être représentées à l'observateur sur le dispositif d'affichage (1.4),
e un ophtalmoscope factice (1.1) et une tête de patient factice (2) étant prévus,
e1 et la tête de patient factice (2) présentant au moins une surface périphérique (2.4) reproduisant la forme d'un visage avec une position d'yeux de patient (2.1) y étant placée, sur laquelle la main peut être appuyée,
f la position spatiale et la situation de l'ophtalmoscope factice (1.1) et de la tête de patient factice (2) pouvant être déterminées au moyen du système de reconnaissance de position (5)
g une partie d'image (2.2) pouvant être incorporée à l'image (1.3) de l'environnement réel (7) représentée à l'observateur,
h - la position spatiale et la situation de l'ophtalmoscope factice (1.1) et de la tête de patient factice (2) et de la fixation (1.5) pouvant être déterminées au moyen du système de reconnaissance de position (5),
i et en fonction de la situation relative de ces trois objets (1.1, 1.2, 1.5)
i1 une image (1.3) de l'environnement réel (7) générée par l'au moins une caméra vidéo concernant la tête de patient factice et l'ophtalmoscope factice (1.1) étant représentée sur le dispositif d'affichage (1.4), et
i2 la partie d'image (2.2) étant incorporée à l'image (1.3) de l'environnement réel (7) représentée à la place de la lentille d'ophtalmoscope factice (1.1), la partie d'image incorporée (2.2) représentant une rétine virtuelle et/ou un environnement virtuel (7), comme l'observateur les verraient par l'ophtalmoscope tenu par lui s'il s'agissait d'un instrument correspondant,
i3 - la partie d'image incorporée (2.2) ne représentant la rétine virtuelle que lorsque la situation relative entre la fixation (1.5) et l'ophtalmoscope factice (1.1) et la tête de patient factice (2) correspond à des conditions réalistes prédéfinies ;
k l'ophtalmoscope factice (1.1) étant un élément factice pour une lentille (1.1) d'un ophtalmoscope indirect ou un élément factice pour un ophtalmoscope direct.

2. Procédé selon la revendication 1,
**caractérisé en ce que**
l'image vidéo (1.3) de l'environnement réel (7), comme la tête de patient factice (2), la main (3.2) de l'observateur, l'ophtalmoscope factice (1.1) et/ou une partie de ceux-ci, est enregistrée au moyen de la caméra vidéo (1.2) et est représentée sur le dispositif d'affichage (1.4).

3. Procédé selon la revendication 1 ou 2,
**caractérisé en ce qu'**
une distance a1 entre la fixation (1.5) et la position des yeux du patient (2.1) et/ou la distance a2 entre l'ophtalmoscope factice (1.1) et la position des yeux du patient (2.1) et/ou la distance a3 entre la fixation (1.5) et la lentille d'ophtalmoscope factice (1.1) et/ou un écart de la distance respective a1, a2, a3 d'une valeur prescrite est/sont affichée(s) pour l'observateur.

4. Procédé selon l'une quelconque des revendications 1 à 3,
**caractérisé en ce que**
des images sont calculées pour la situation et/ou le mouvement de la fixation (1.5) et/ou de l'ophtalmoscope factice (1.1), lesquelles reproduisent une vue à partir d'au moins un autre angle de vision dans l'espace.

5. Procédé selon l'une quelconque des revendications 1 à 4,
**caractérisé en ce que**
des mouvements d'yeux du patient sont simulés.
